# EUROPEAN PATENT APPLICATION

(11) **EP 4 624 453 A1**
(43) Date of publication of application: **01.10.2025**
(21) Application number: 23893808.8
(22) Date of filing: 21.11.2023
(51) Int. Cl.: C07C 319/20, C07C 15/04, C07C 317/24, C07C 315/04

(54) **METHOD FOR PREPARING CYCLOSULFONONE, AND INTERMEDIATES**

(30) Priority: 22.11.2022 CN 202211487542
(71) Applicant: Lansheng Biotechnology Group Co., Ltd, Shijiazhuang, Hebei 052260 (CN); Hebei University of Science and Technology, Shijiazhuang, Hebei 050018 (CN)
(72) Inventor: LI, Liuliu, Shijiazhuang, Hebei 050018 (CN); ZHAO, Yaran, Shijiazhuang, Hebei 050018 (CN); GUO, Qingchun, Shijiazhuang, Hebei 052260 (CN); SONG, Haiwen, Shijiazhuang, Hebei 052260 (CN); ZHANG, Yue, Shijiazhuang, Hebei 050018 (CN)
(74) Representative: karo IP
(86) International application number: PCT/CN2023/132821
(87) International publication number: WO 2024/109718

(57) **Abstract**

Provided is a method for preparing cyclosulfonone, which is characterized by comprising any one of the following steps.

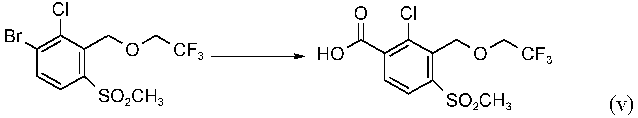

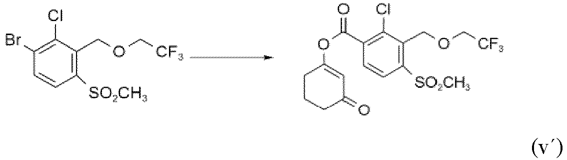

## Description

### Technical Field

The present invention relates to a novel method for preparing the herbicide cyclosulfonone and new intermediate compounds used therein.

### Background Art

Cyclosulfonone is a triketone herbicide with advantages such as high efficacy, low toxicity, minimal residue, and broad-spectrum activity. Therefore, its market has been expanding since its launch.

As a method for preparing cyclosulfanone, the patent DE19846792A1 discloses the following method:

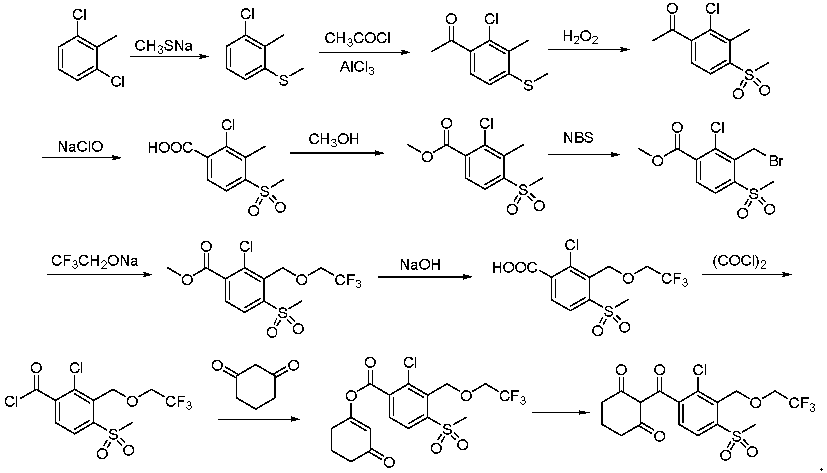

In the above method, the raw material dichlorotoluene has poor selectivity during the reaction, resulting in high impurities and low yield.

Currently, the following route is widely reported and adopted (see, for example, Chinese Patent Application No. 200510030163.5),

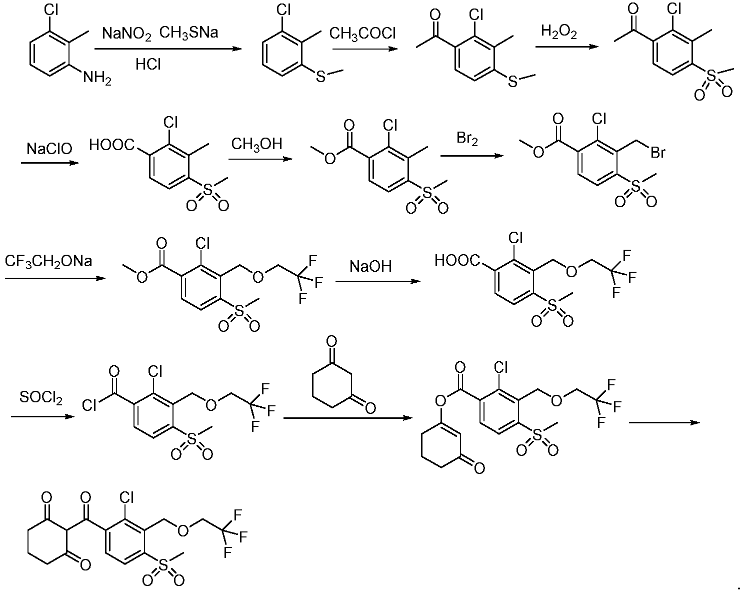

However, the above method has 11 steps in total, which is overly lengthy; The Friedel-Crafts (F-C) reaction employing acetyl chloride is highly irritating, and the post-treatment produces a large amount of wastewater containing aluminum trichloride catalyst, and the later sodium hypochlorite haloform reaction also occurs in the aqueous phase, which generates haloform and produces a large amount of wastewater containing sodium chloride, which is highly polluting; Additionally, after introducing the acetyl group in the molecular via the F-C reaction, a subsequent chloroform reaction was required to cleave the methyl group, which is unreasonable from the aspect of raw material economy, and there are problems such as high cost and low efficiency.

### Summary of the Invention

The present invention aims to provide a novel method for preparing cyclosulfonone and new intermediate compounds used therein.

Through the new method and new intermediates of the present invention, cyclosulfanone can be prepared in fewer steps than the existing process route described above, saving costs and improving productivity.

Specifically, the present invention provides:
(1) Method for preparing cyclosulfonone, which is characterized by comprising any one of the following steps: 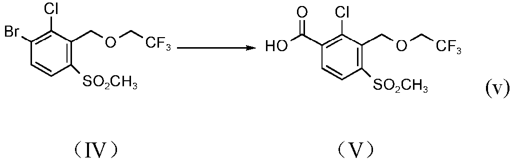 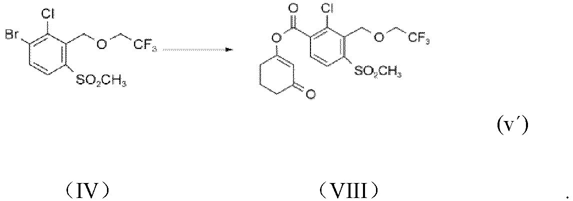
(2) The method according to above (1), wherein steps (v) and (v') are respectively the following steps: 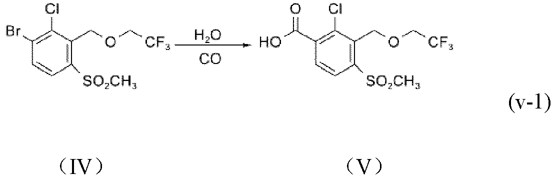 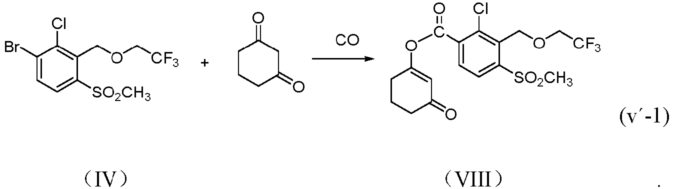
(3) The method according to above (1) or (2), further comprising any one or more of the intermediate preparation steps (i) ~ (iv) as shown below: 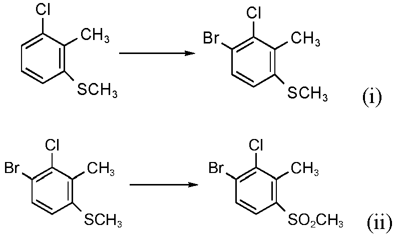 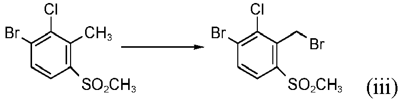 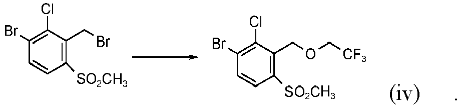
(4) The method according to above (3), wherein steps (i) to (iv) are independently the following steps: 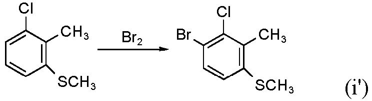 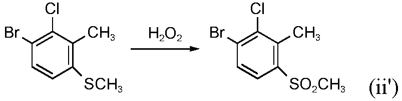 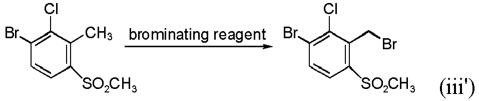 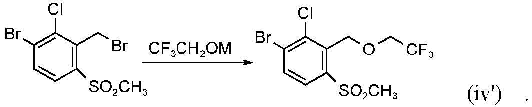 In step (iii'), the brominating reagent is bromine, N-bromosuccinimide, or a combination of hydrogen bromide and hydrogen peroxide, preferably bromine; In step (iv'), CF₃CH₂OM is CF₃CH₂ONa or CF₃CH₂OK.
(5) The method according to any one of above (1) to (4), comprising the following intermediate preparation steps: 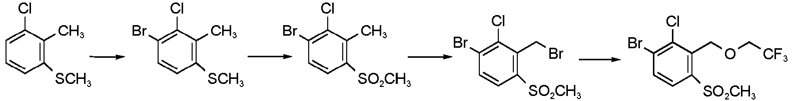
(6) The method according to above (5), wherein the intermediate preparation steps are as follows: 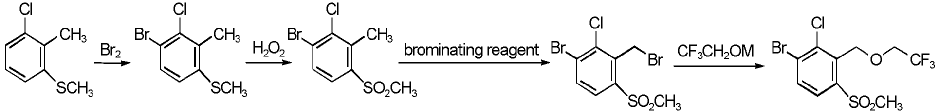 In the formula, the brominating reagent is bromine, N-bromosuccinimide, or a combination of hydrogen bromide and hydrogen peroxide, preferably bromine; CF₃CH₂OM is CF₃CH₂ONa or CF₃CH₂OK.
(7) The method according to above (5) or (6), further comprising the following steps: 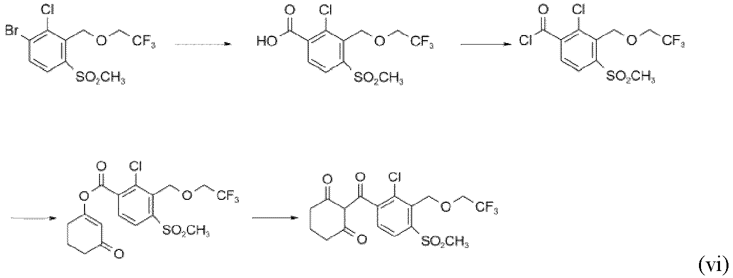 or 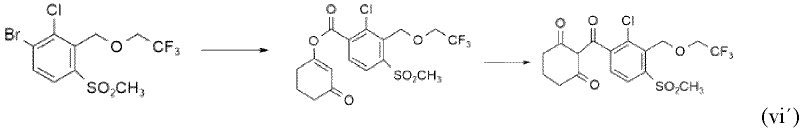 wherein Step (vi') is carried out in a stepwise way with isolation of the intermediate ester compound or in a one-pot way without isolation of the intermediate ester compound.
(8) The method according to above (7), wherein steps (vi) and (vi') are respectively the following steps: 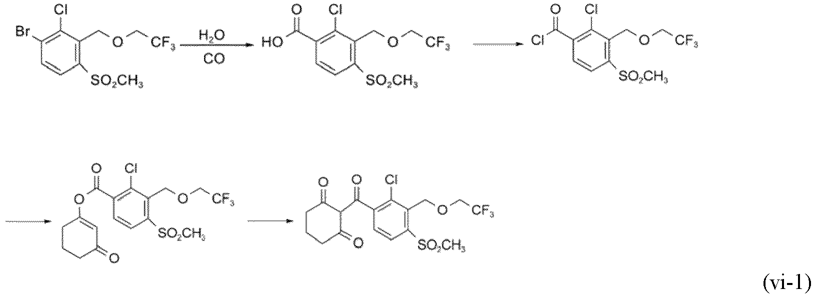 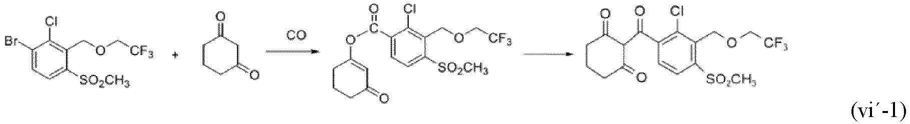 wherein Step (vi'-1) is carried out in a stepwise way with isolation of the intermediate ester compound or in a one-pot way without isolation of the intermediate ester compound.
(9) The compound shown in formula (A) below: 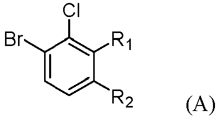 wherein R₁ and R₂ are any one of the following:
   R₁ is CF₃-CH₂-O-CH₂-, and R₂ is CH₃-SO₂-;
   R₁ is Br-CH₂-, and R₂ is CH₃-SO₂-;
   R₁ is CH₃-, and R₂ is CH₃-SO₂-;
   R₁ is CH₃-, and R₂ is CH₃-S-.
(10) Method for preparing cyclosulfonone, which is characterized by using the compound according to above (9) as an intermediate.
(11) Use of the compound according to above (9) as an intermediate for preparing cyclosulfanone.

### Description of the Drawings

Figure 1 shows the ¹HNMR spectrum of the compound of formula (VI) prepared in Example 1.
Figure 2 shows the ¹³CNMR spectrum of the compound of formula (VI) prepared in Example 1.
Figure 3 shows the ¹HNMR spectrum of the compound of formula (I) prepared in Example 2.
Figure 4 shows the ¹³CNMR spectrum of the compound of formula (I) prepared in Example 2.
Figure 5 shows the ¹HNMR spectrum of the compound of formula (II) prepared in Example 3.
Figure 6 shows the ¹³CNMR spectrum of the compound of formula (II) prepared in Example 3.
Figure 7 shows the ¹HNMR spectrum of the compound of formula (III) prepared in Example 4.
Figure 8 shows the ¹³CNMR spectrum of the compound of formula (III) prepared in Example 4.
Figure 9 shows the ¹HNMR spectrum of the compound of formula (IV) prepared in Example 5.
Figure 10 shows the ¹³CNMR spectrum of the compound of formula (IV) prepared in Example 5.
Figure 11 shows the ¹HNMR spectrum of the compound of formula (V) prepared in Example 6.
Figure 12 shows the ¹³CNMR spectrum of the compound of formula (V) prepared in Example 6.
Figure 13 shows the ¹HNMR spectrum of the compound of formula (VIII) prepared in Example 9.
Figure 14 shows the ¹³CNMR spectrum of the compound of formula (VIII) prepared in Example 9.
Figure 15 shows the ¹HNMR spectrum of the compound of cyclosulfanone prepared in Example 10.
Figure 16 shows the ¹³CNMR spectrum of the compound of cyclosulfanone prepared in Example 10.

### Detailed Description of the Invention

In one aspect, the present invention provides a new compound shown in formula (A) below: 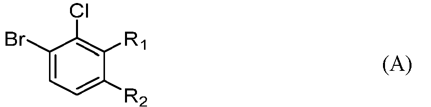 wherein R₁ and R₂ are any one of the following:
R₁ is CF₃-CH₂-O-CH₂-, and R₂ is CH₃-SO₂-;
R₁ is Br-CH₂-, and R₂ is CH₃-SO₂-;
R₁ is CH₃-, and R₂ is CH₃-SO₂-;
R₁ is CH₃-, and R₂ is CH₃-S-.

The compound shown in formula (A) specifically includes compounds of formula (I)-(IV) as follows:

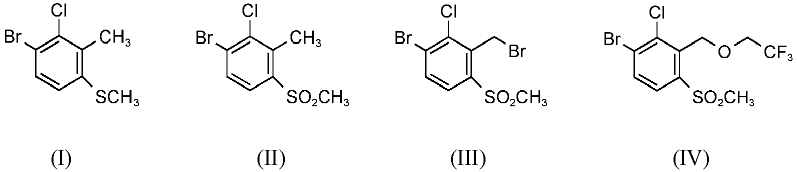

The compound of formula (I) of the present invention can be prepared by the following route:

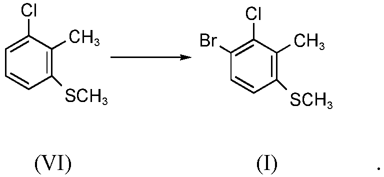

In specific embodiments, the above route is carried out using compound (VI) and bromine as raw materials, as follows, but not limited to this:

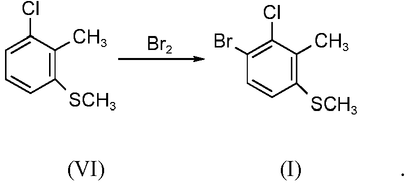

In more specific embodiments, the above reaction is performed as follows but not limited to this: Bromine is added dropwise to compound (VI). After completion of the reaction, the mixture is quenched with a basic solution, washed sequentially with a basic solution and water, separated the solution to obtain the compound (I). Specific examples of the basic solution include, but not limited to, aqueous NaOH solution.

In more specific embodiments, the above reaction is carried out at a temperature of approximately 10~30°C.

In more specific embodiments, in the above reaction dichloromethane or 1,2-dichloroethane is used as a solvent.

The compound of formula (VI) as a raw material can be prepared by the following reaction with reference to, for example, the well-known method described in patent document CN106631941A and the like:

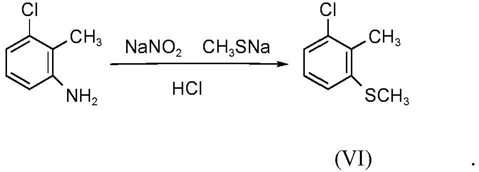

The compound of formula (II) of the present invention can be prepared by the following route:

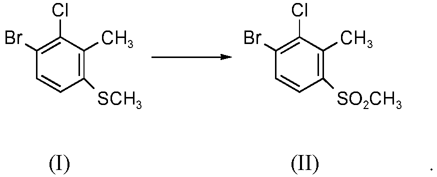

In specific embodiments, the above route is carried out using hydrogen peroxide as the oxidizing agent, as follows, but not limited to this:

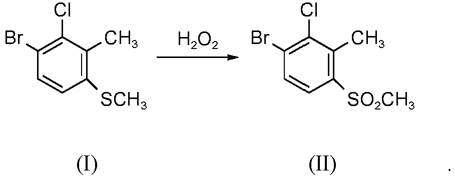

In more specific embodiments, the above reaction is performed as follows but not limited to this: compound (I) is mixed with a solvent and optionally a catalyst. The mixture is heated, and hydrogen peroxide is added dropwise. After completion of the reaction, the mixture is cooled, filtered, washed with water, and dried to obtain compound (II).

In more specific embodiments, tungstate, metatungstate, vanadate or metavanadate is used as a catalyst in the above reaction. The salt includes sodium, potassium or ammonium salt. Preferably, sodium tungstate dihydrate is used as the catalyst.

In more specific embodiments, glacial acetic acid or formic acid is used as a solvent in the above reaction.

In more specific embodiments, the temperature is raised to 40~100°C, preferably to 70~90°C in the above reaction.

In more specific embodiments, the molar ratio of the compound (I) to hydrogen peroxide in the above reaction is approximately 1~5, preferably 2~3.

The compound of formula (III) of the present invention can be prepared by the following route:

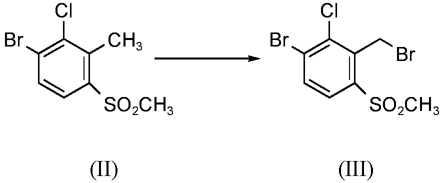

In specific embodiments, the above route is carried out using compound (II) and brominating reagent as raw materials, as follows, but not limited to this:

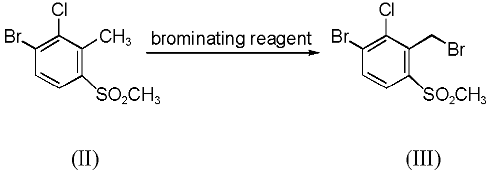

In more specific embodiments, the above reaction is performed as follows but not limited to this: compound (II) is mixed with a solvent and optionally an initiator. The mixture is heated, and a brominating reagent is added dropwise. After completion of the reaction, the mixture is quenched with a basic solution, washed sequentially with a basic solution and water, the solvent is removed by evaporation. The resulting solid is dried to obtain compound (III). Specific examples of the basic solution include, but not limited to, aqueous NaOH solution.

In more specific embodiments, bromine, N-bromosuccinimide, or a combination of hydrogen bromide and hydrogen peroxide is used as the brominating reagent in the above reaction, preferably bromine is used as the bromine reagent.

In more specific embodiments, benzoyl peroxide (BPO), azobisisobutyronitrile (AIBN) are used as initiators in the above reaction.

In more specific embodiments, dichloromethane, dichloroethane, or carbon tetrachloride is used as a solvent in the above reaction.

In more specific embodiments, the temperature is raised to 40-100°C, preferably to 50-80°C in the above reaction.

The compound of formula (IV) of the present invention can be prepared by the following route:

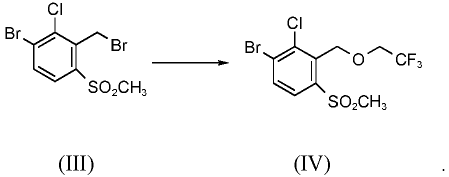

In specific embodiments, the above route is carried out using compound (III) and sodium trifluoroethanol or potassium trifluoroethanol as raw materials, as follows, but not limited to this.

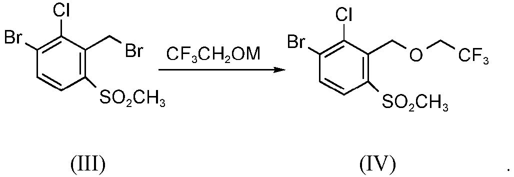

In above formula, CF₃CH₂OM is CF₃CH₂ONa or CF₃CH₂OK.

In more specific embodiments, the above reaction is performed as follows but not limited to this: compound (III) and sodium trifluoroethanol or potassium trifluoroethanol are dissolved in a solvent separately. The solution of compound (III) is added dropwise to the solution of sodium trifluoroethanol or potassium trifluoroethanol. After completion of the reaction, the mixture is quenched with water, separated, washed the organic layer with saturated brine, evaporated the solvent, recrystallized with methanol or ethanol and dried to obtain compound (IV).

In more specific embodiments, tetrahydrofuran, 1,4-dioxane, or ether is used as a solvent in the above reaction, and preferably anhydrous tetrahydrofuran is used as a solvent.

In more specific embodiments, when the solution of compound(III) is added dropwise to the solution of sodium trifluoroethanol or potassium trifluoroethanol, the reaction temperature during dropwise addition is controlled not to exceed -5~15 °C.

In more specific embodiments, the sodium trifluoroethanol or potassium trifluoroethanol is prepared by reacting sodium or potassium hydride, sodium or potassium methoxide, sodium or potassium hydroxide with trifluoroethanol.

The compound of formula (IV) of the present invention can be used to prepare the carboxylic acid compound of formula (V) through, but not limited to, the following carbonylation reaction:

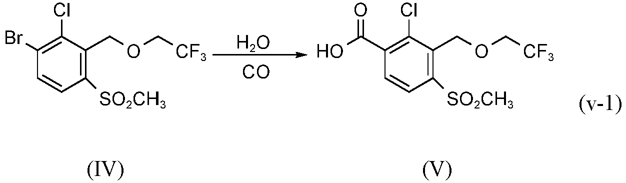

In more specific embodiments, the above reaction (v-1) is performed as follows but not limited to this: add the compound of formula (IV), water, acid binding agent, catalyst, ligand and a first solvent into a high-pressure autoclave, evacuate the system and replace with nitrogen, introduce CO, and react at 80~120°C, preferably 80~90°C for 10~20 hours. Filter, evaporate the first solvent, then dissolve the residue in a second solvent, adjust the pH to 2~3 with acid to precipitate a white solid, and filter to obtain the compound of Formula (V).

In more specific embodiments, the catalyst used in the above reaction is a noble metal catalyst such as palladium, rhodium, ruthenium, copper, cobalt, or the like.

In more specific embodiments, a palladium catalyst is used. Specific examples include, but not limited to, palladium chloride (PdCl₂), palladium carbonate (PdCO₃), palladium acetate[Pd(OAc)₂], palladium/carbon (Pd/C), palladium trifluoroacetate [Pd(OTFA)₂], palladium bis(dibenzylideneacetone) [Pd(dba)₂], and the like, preferably palladium chloride (PdCl₂).

In more specific embodiments, a phosphorus ligand or salt thereof is used as a catalyst ligand in the above reaction, preferably 1,3-bis(diphenylphosphino)propane, 1,4-bis(diphenylphosphino)butane, or bis[(4-N,N-dimethylamino)phenyl]di-tert-butylphosphine, or the like.

In more specific embodiments, the acid binding agent used in the above reaction is triethylamine, potassium carbonate, sodium carbonate, cesium carbonate (CsCO₃), potassium phosphate, N,N-dimethylacetamide (DMAC), N,N-diisopropylethylamine (DIPEA), 1,8-diazabicyclohexadec-7-ene (DBU), triethylenediamine (DABCO), or the like.

In more specific embodiments, the first solvent used in the above reaction is a solvent selected from aromatic hydrocarbons, sulfoxides, ethers, or amides, preferably one or two or more of toluene, 1,4-dioxane, ethylene glycol dimethyl ether, ethylene glycol diethyl ether, dimethyl sulfoxide (DMSO), tetrahydrofuran (THF), N,N-dimethylformamide (DMF).

In more specific embodiments, the pressure of CO introduced during the above reaction is approximately 1~4 MPa.

In more specific embodiments, in the above reaction, after evaporating the first solvent, an alcohol solvent such as methanol or ethanol is added as the second solvent for dissolution.

In other embodiments, the compound of formula (IV) of the present invention can be used directly to prepare the ester compound of formula (VIII) through, but not limited to, the following carbonylation reaction:

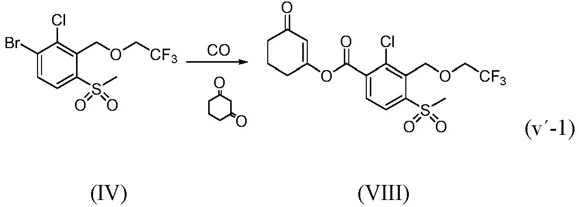

The carboxylic acid compound of formula (V) and the ester compound of formula (VIII) are important intermediates for the preparation of cyclosulfonone, which can be used to prepare cyclosulfonone by known methods disclosed in, for example, patent literature DE19846792A1, through the following route. When preparing cyclosulfonone by rearrangement of the ester compound of formula (VIII), it is preferred to use a cyanide catalyst, particularly acetone cyanohydrin as the catalyst.

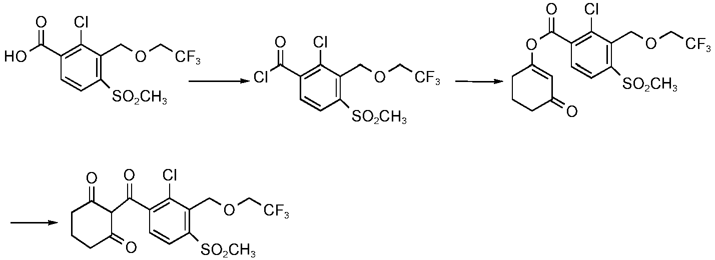

In more specific embodiments, the implementation method of the above reaction (v'-1) is the same as that of the previously described reaction (v-1), except that no water is added to the reactants.

After preparing the intermediate ester compound of formula (VIII) from the compound of formula (IV), the intermediate ester compound can be separated first to prepare cyclosulfonone stepwise. Alternatively, cyclosulfonone can be directly prepared by a one-pot method without separating the intermediate ester compound.

In the above reactions of (v-1) and (v'-1), the method of carbonylation via CO insertion is exemplified, but the present invention is not limited thereto. Other methods for preparing the intermediate acid compound of formula (V) or directly preparing the intermediate ester compound of formula (VIII) from the compound of formula (IV) are also included within the scope of the present invention.

In another aspect, the present invention provides a new method for preparing cyclosulfonone, which includes using any one or two or more compound(s) of formula (I) to formula (IV) as intermediates. Regardless of whether these intermediate compounds are separated during the preparation process, the use of these intermediates in the form of individual forms or in the form of an unseparated mixture for the preparation of cyclosulfonone falls within the scope of the new method of the present invention.

A further aspect of the present invention provides a new method for preparing cyclosulfonone, which includes any one of the following steps:

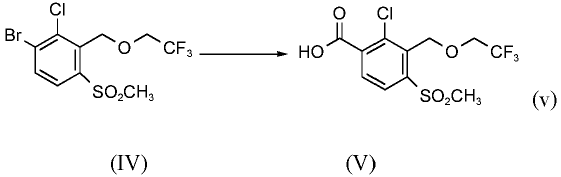

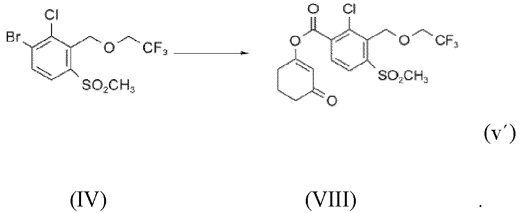

Wherein, route (v) represents the preparation of the carboxylic acid compound of formula (V) from the compound of formula (IV), and route (v') represents the direct preparation of the ester compound of formula (VIII) from the compound of formula (IV).

In more specific embodiments, the steps (v) and (v') are respectively the following steps, and the more specific implementation methods of these steps are described as previously.

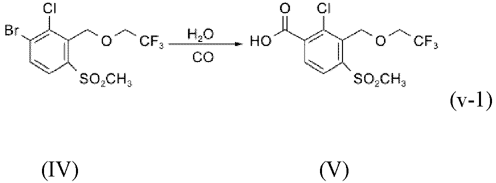

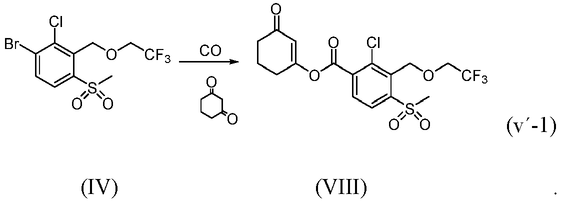

The new method for preparing cyclosulfonone of the present invention can further include any one or two or more of the following intermediate preparation step(s) (i) to (iv):

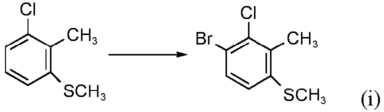

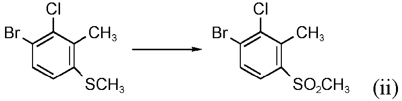

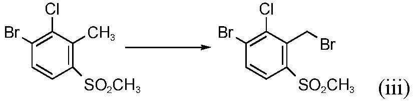

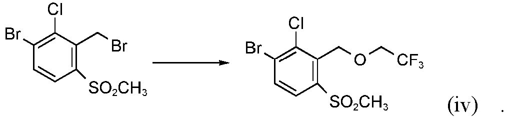

In specific embodiments, the above steps (i) to (iv) may be the following steps (i') to (v'). The more specific implementation methods of these steps are described as previously.

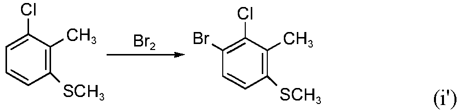

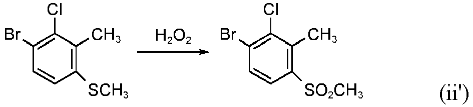

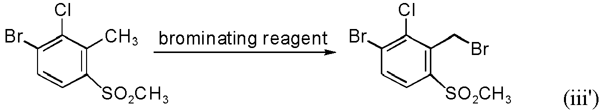

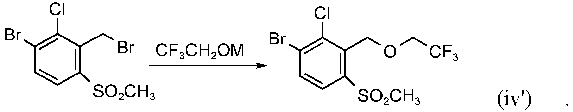

In more specific embodiments, the new method of the present invention includes the following steps: 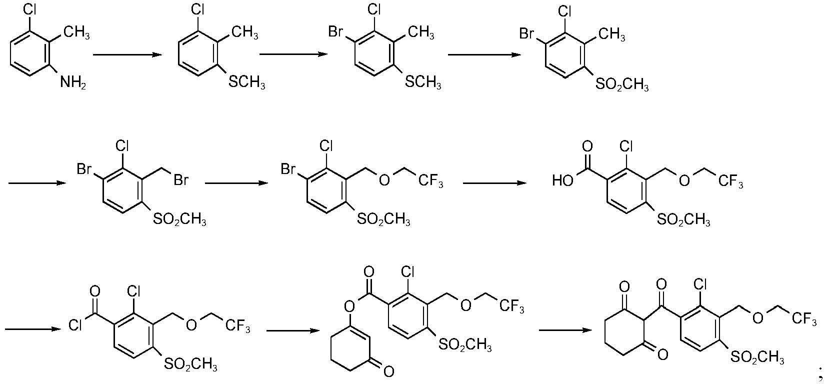 or

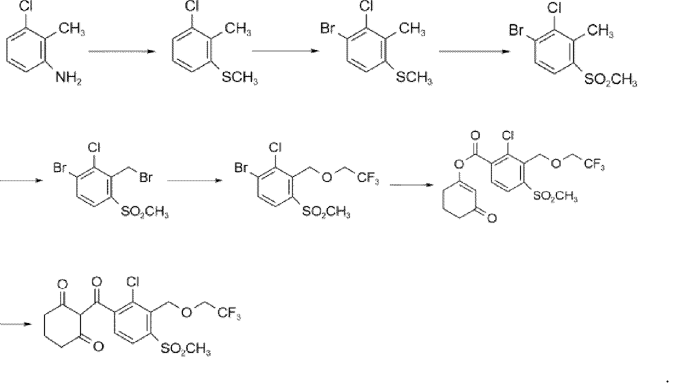

In more specific embodiments, the new method of the present invention includes the following steps: 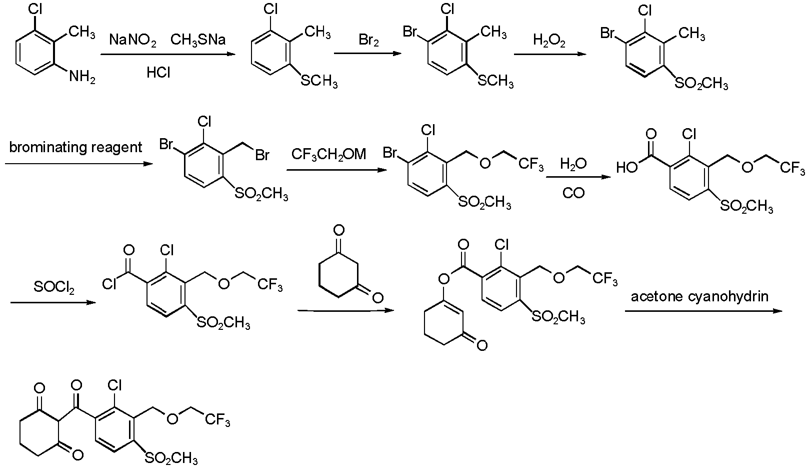 or

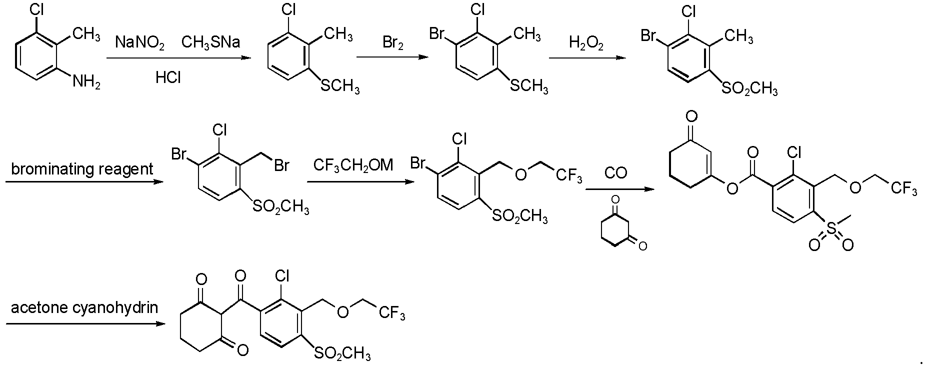

In the formula, brominating reagent is bromine, N-bromosuccinimide, or a combination of hydrogen bromide and hydrogen peroxide, preferably bromine; CF₃CH₂OM is CF₃CH₂ONa or CF₃CH₂OK.

In a further aspect, the present invention provides the use of the compounds of formulas (I) to (IV) as intermediates for the preparation of cyclosulfonone.

### Examples

### Example 1 Preparation of compound of formula (VI)

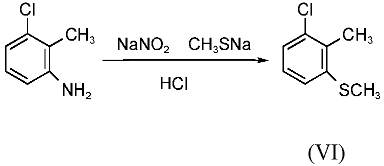

1165g of 5.1% hydrochloric acid was added to a four-necked flask at 20°C, 100g of 3-chloro-2-methylaniline was added dropwise. After the dropwise addition was completed, 251.41g of 20% NaNO₂ aqueous solution was further added dropwise at 0°C, the temperature was controlled to be no more than -5~10°C during the dropwise addition process, stirred to obtain the diazonium salt solution. 273g of 20% CH₃SNa aqueous solution and 26.62g of 32% NaOH aqueous solution were weighed in another four-necked flask. The above diazonium salt solution was added dropwise into this flask, and the reaction was monitored by HPLC. After completion of the reaction, the pH was adjusted to 2~3 with hydrochloric acid, layered, collected the organic layer, and distilled to obtain the compound of formula (VI).

HPLC conditions: Kromasil C18 reverse-phase column, mobile phase: acetonitrile: water (pH adjusted to 3.7~4.1 with formic acid) = 80: 20 (V: V), flow rate: 1.0 mL/min, column temperature: 30°C, detection wavelength: 210 nm, injection volume: 5 µL.

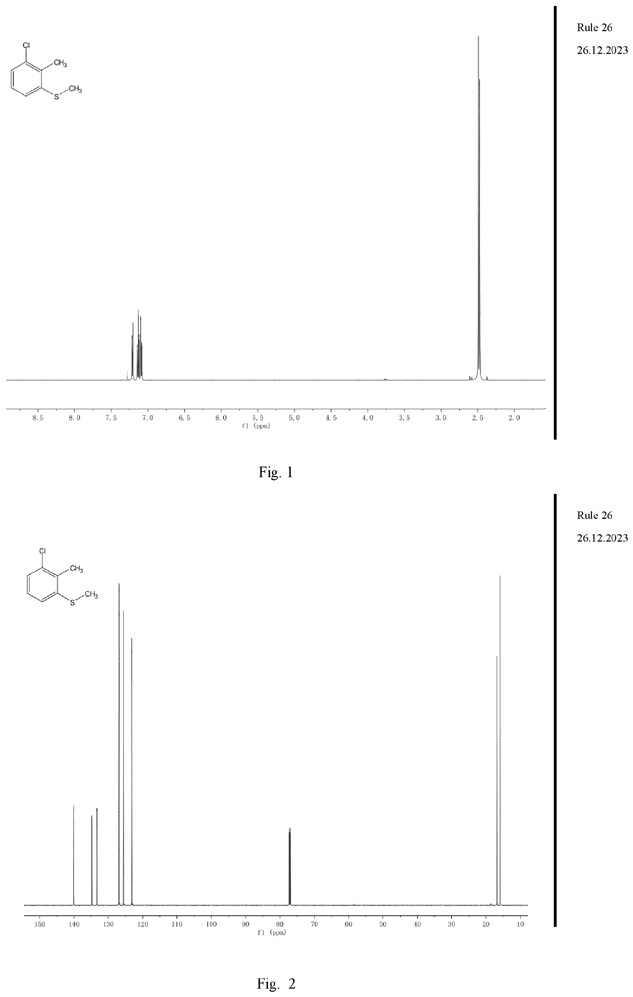

The ¹HNMR (CDCl₃) spectrum of the compound of formula (VI) is shown in Figure 1, and the ¹³CNMR (CDCl₃) spectrum is shown in Figure 2.

### Example 2 Preparation of compound of formula (I)

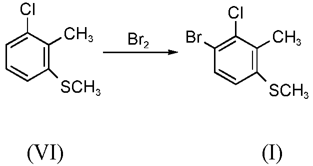

100.75g of compound of formula (VI) and 400g of dichloromethane were placed in a four-necked round-bottomed flask, and 98.4g of bromine was added dropwise at about 10°C. After confirming the completion of the reaction by HPLC monitoring, the reaction was quenched with 30 mL of 32% NaOH solution, the organic phase was separated, and washed twice with 70 mL of 32% NaOH aqueous, then twice with 70 mL of water and separated, the organic layer was rotary evaporated to remove solvent to obtain the compound of formula (I).

HPLC conditions: The detection wavelength was 254 nm, and other conditions were the same as described in Example 1.

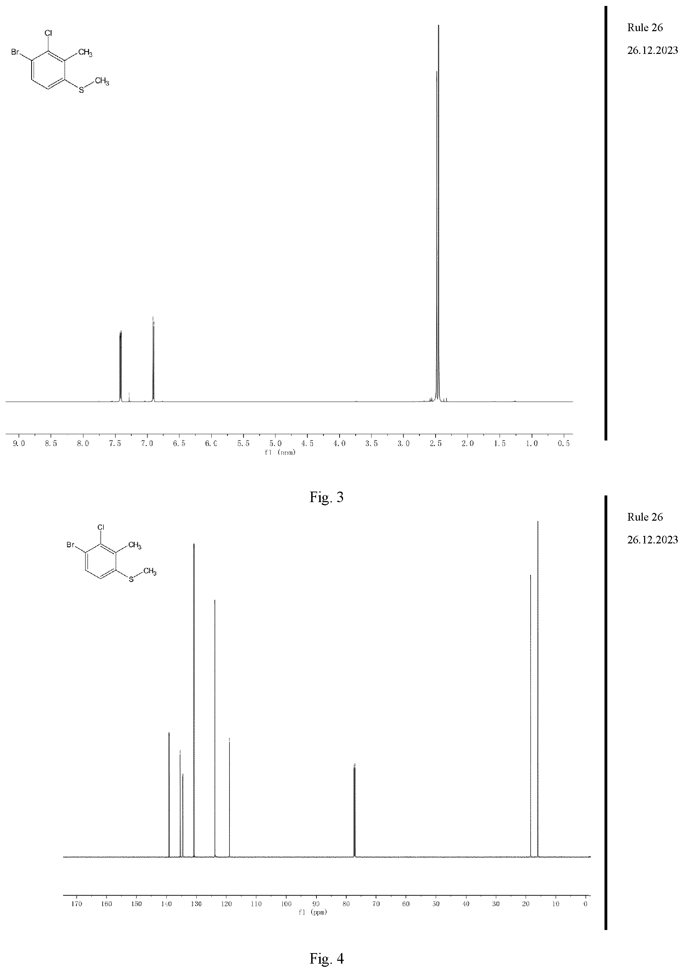

The ¹HNMR (CDCl₃) spectrum of the compound of formula (I) is shown in Figure 3, and the ¹³CNMR (CDCl₃) spectrum is shown in Figure 4.

### Example 3 Preparation of compound of formula (II)

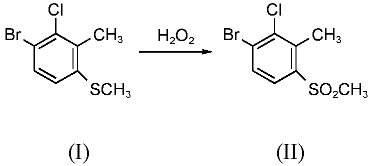

146g of the compound of formula (I), 1.91g of sodium tungstate dihydrate, and 600 mL of glacial acetic acid were added to a four-necked flask, heated to 80°C, and 224g of 23% hydrogen peroxide was added dropwise. After confirming the completion of the reaction by HPLC monitoring, the reaction was stopped, cooled, and filtered. The filter cake was washed twice with water, then dried at 60°C to obtain the compound of formula (II). Melting point: 138~140°C.

HPLC conditions: same as in Example 1.

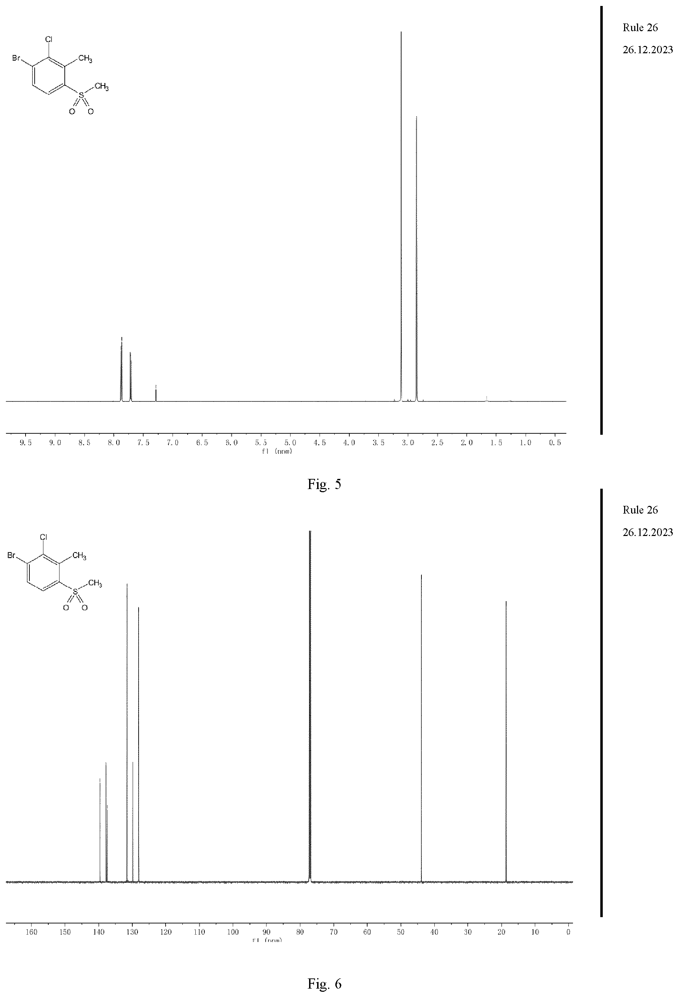

The ¹HNMR (CDCl₃) spectrum of the compound of formula (II) is shown in Figure 5, and the ¹³CNMR (CDCl₃) spectrum is shown in Figure 6.

### Example 4 Preparation of compound of formula (III)

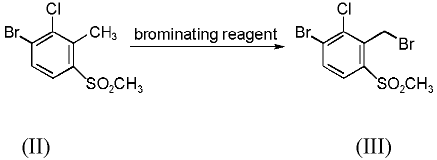

**Method 1:** 84g of the compound of formula (II), 3.58g of benzoyl peroxide, and 500 mL of 1,2-dichloroethane were added to a four-necked round-bottomed flask, heated to 60°C, and 52.8g of bromine was added dropwise. After the dropwise addition of bromine was completed, and confirming the completion of the reaction by HPCL monitoring, the reaction was quenched with 50 mL of a 32% NaOH aqueous solution, separated the organic phase, washed twice with 50 mL of a 32% NaOH aqueous solution, and twice with 50 mL of water. The solvent was removed by rotary evaporation at 50°C, and the resulting solid was dried at 60°C to obtain the compound of formula (III). Melting point: 148~150°C.

**Method 2:** 84g of the compound of formula (II), 3.58g of benzoyl peroxide, 64.1g of N-bromosuccinimide, and 500 mL of carbon tetrachloride were added to a four-necked round-bottomed flask, heated to 60°C. After confirming the completion of the reaction by HPLC monitoring, filtered, the filtrate was rotary evaporated at 50°C to remove the solvent, and the residue was recrystallized with 150 mL of ethanol, filtered and the filter cake was dried at 60°C to obtain the compound of formula (III).

HPLC conditions: same as in Example 1.

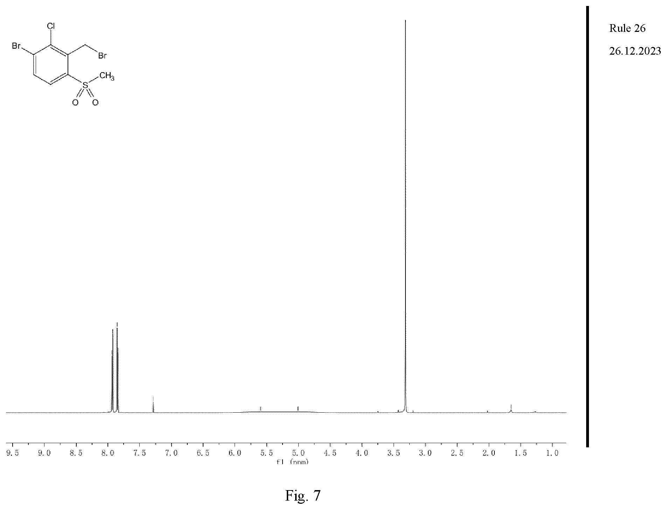

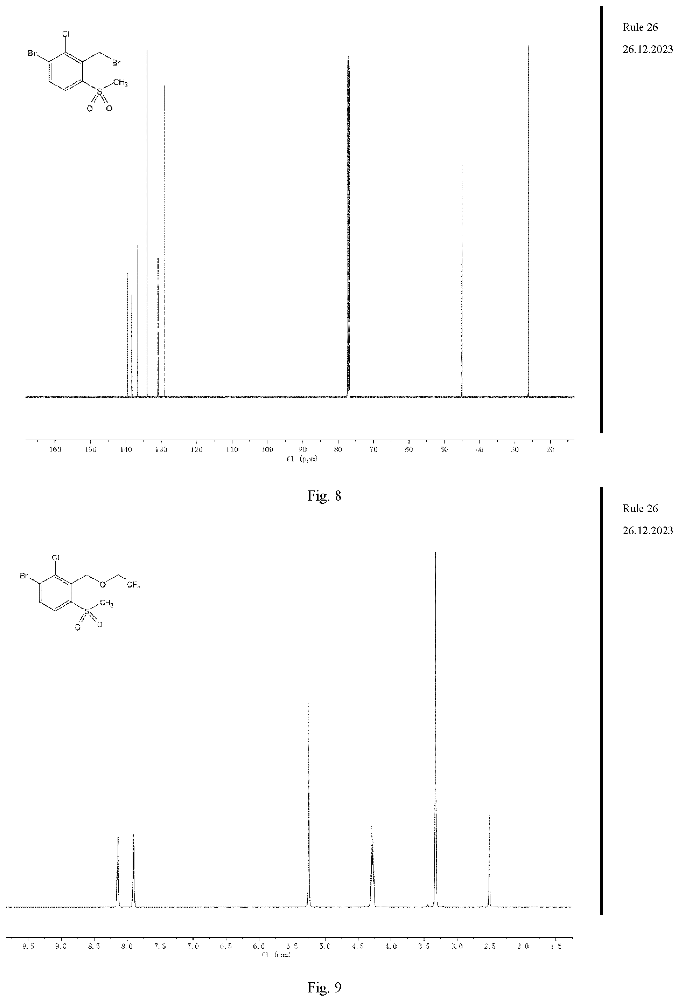

The ¹HNMR (CDCl₃) spectrum of the compound of formula (III) is shown in Figure 7, and the ¹³CNMR (CDCl₃) spectrum is shown in Figure 8.

### Example 5 Preparation of compound of formula (IV)

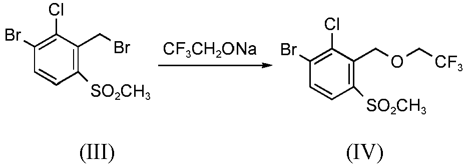

9.2g of 60% sodium hydride was dissolved in 80 mL of anhydrous tetrahydrofuran at -5 to 5°C, 24g of trifluoroethanol in 20 mL of anhydrous tetrahydrofuran was added dropwise to this solution. After completion of the dropwise addition, 83g of the compound of formula (III) in 400 mL of anhydrous tetrahydrofuran was further added dropwise under the condition that the reaction temperature was controlled to not exceed 15°C. After the dropwise addition was completed, stirred and the reaction was quenched with water after confirming the completion of the reaction by HPLC monitoring. Separated the liquid, the organic phase was washed twice with 40 mL of saturated brine, the solvent was removed by rotary evaporation, and the residue was recrystallized with 120g of methanol or ethanol. Filtered and the filter cake was dried at 60°C to obtain the compound of formula (IV). Melting point: 80~82°C.

HPLC conditions: Kromasil C18 reversed-phase column, mobile phase: acetonitrile: water (with 0.1% formic acid) = 60: 40 (V: V), flow rate: 1.0 mL/min, column temperature: 30°C, detection wavelength: 210 nm, injection volume: 5 µL.

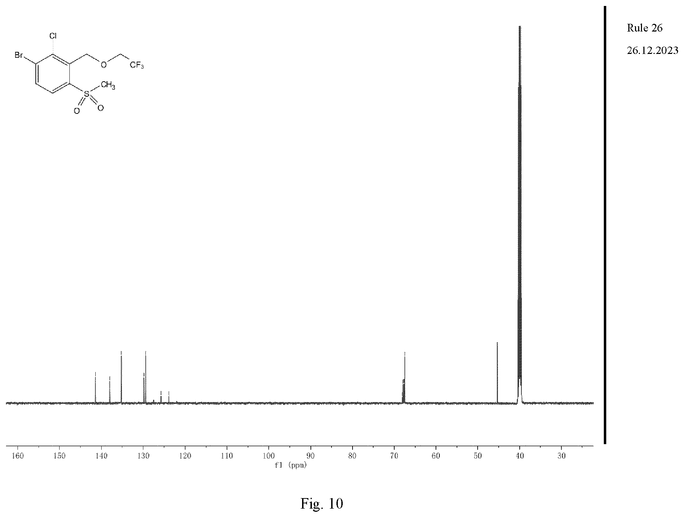

The ¹HNMR (DMSO) spectrum of the compound of formula (IV) is shown in Figure 9, and the ¹³CNMR (DMSO) spectrum is shown in Figure 10.

### Example 6 Preparation of carboxylic acid compound of formula (V)

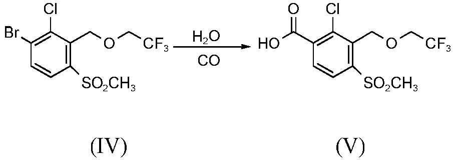

40g of the compound of formula (IV), 20g of water, 26.49g of triethylamine, 0.4g of PdCl₂ catalyst, 4.65g of 1,4-bis(diphenylphosphino)butane ligand, and 400g of 1,4-dioxane solvent were added to a high-pressure reactor. Evacuated and replaced with nitrogen, then introduced CO at a pressure of about 1~4 MPa, and reacted at 80°C for about 12 hours. After confirming the completion of the reaction by HPLC monitoring, filtered, removed the dioxane solvent by rotary evaporation, added methanol to dissolve, adjusted the pH to 2~3 with acid, and precipitated a white solid. Filtered, the filter cake washed with water, slurried and filtered to obtain the compound of formula (V), yield 81.13%, melting point:155~157°C.

HPLC conditions: same as in Example 5.

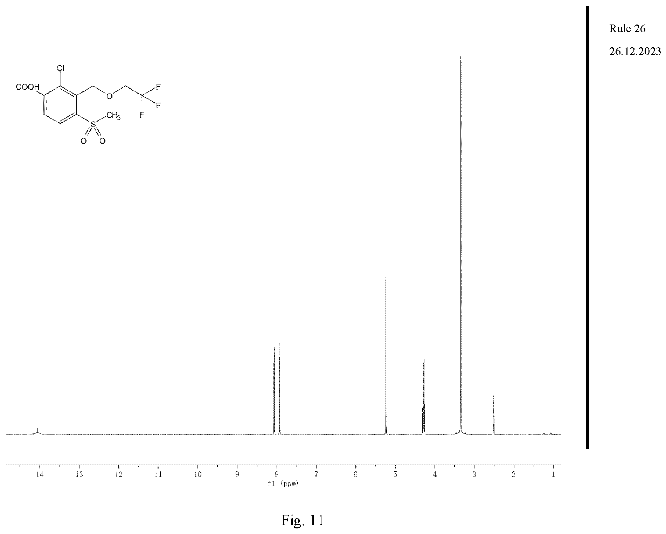

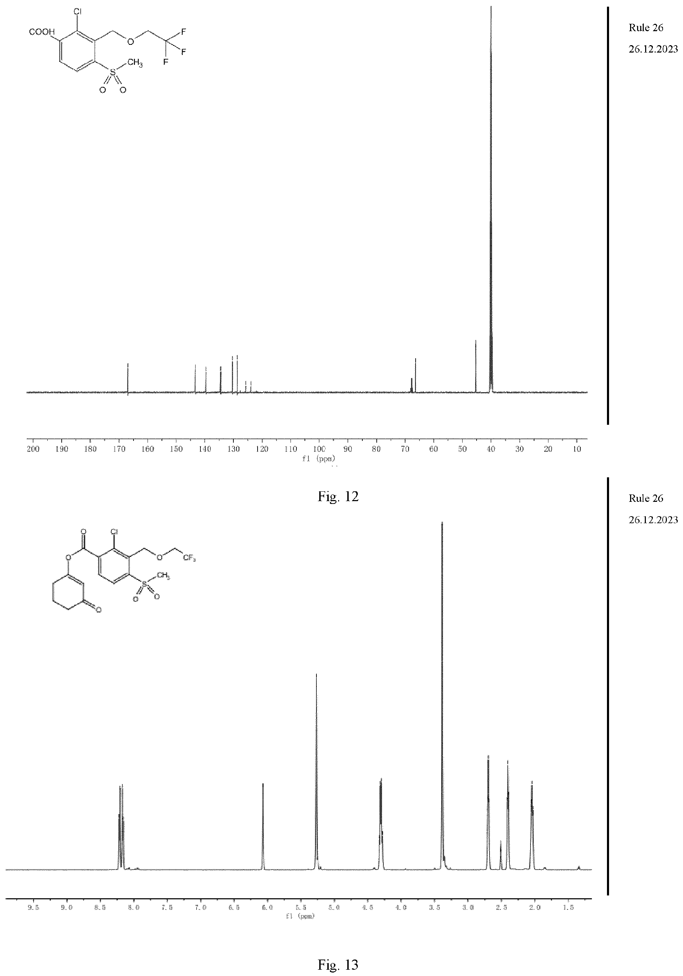

The ¹HNMR (DMSO) spectrum of the compound of formula (V) is shown in Figure 11, and the ¹³CNMR (DMSO) spectrum is shown in Figure 12.

### Example 7 Preparation of carboxylic acid compound of formula (V)

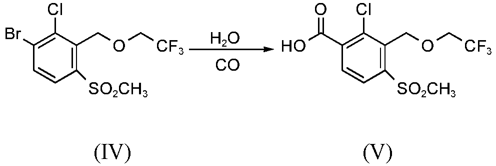

40g of the compound of formula (IV), 20g of water, 26.49g of triethylamine, 0.4g of PdCl₂ catalyst, 3.95g of 1,3-bis(diphenylphosphino)propane ligand, and 400g of tetrahydrofuran solvent were added to a high-pressure reactor. Evacuated and replaced with nitrogen, then introduced CO at a pressure of about 1~4 MPa, and reacted at 80°C for about 12 hours. After confirming the completion of the reaction by HPLC monitoring, filtered, removed the tetrahydrofuran solvent by rotary evaporation, added methanol to dissolve, adjusted the pH to 2-3 with acid, and precipitated a white solid. Filtered, the filter cake washed with water, slurried and filtered to obtain the compound of formula (V), yield 67.57%, melting point: 155~157°C.

### Example 8 Preparation of cyclosulfonone (I)

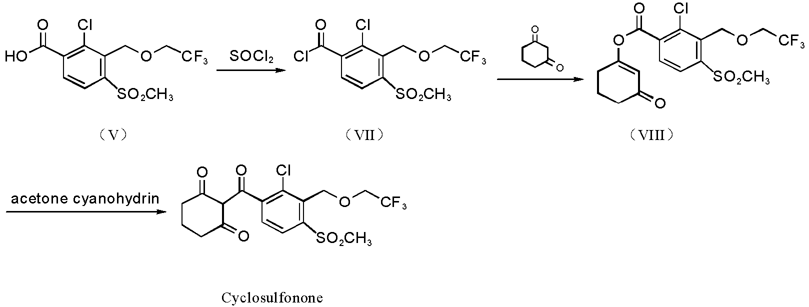

1.5g of the compound of formula (V) was dissolved in 20 mL of 1,4-dioxane, 1.03g of thionyl chloride was added dropwise at 5°C, and heated to 55°C to react. After confirming the completion of the reaction by HPLC monitoring, removed the solvent by rotary evaporation to obtain the compound of formula (VII). 0.5g of 1,3-cyclohexanedione was dissolved in 15mL of dioxane, 0.44g of triethylamine was added, and added dropwise to the above prepared compound of formula (VII) in an ice bath. After the dropwise addition was completed, stirred at room temperature for about 2 hours. After confirming the completion of the reaction by HPLC monitoring, the solvent was removed by rotary evaporation to obtain a solution of the compound of formula (VIII).

0.1 mL of acetone cyanohydrin was added to the above solution of the compound of formula (VIII), reacted at 60°C for 4 hours. After confirming the completion of the reaction by HPLC monitoring, filtered, the filtrate was rotary evaporated to remove the solvent, added water, and adjusted the pH to 2~3 with hydrochloric acid, filtered the resulting solid, and dried to obtain cyclosulfonone, yield 76.59%.

HPLC conditions: same as in Example 5.

### Example 9 Direct preparation of ester intermediate of formula (VIII)

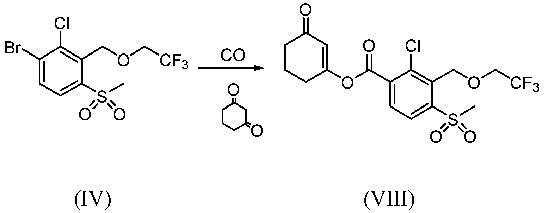

**Method 1:** 94.73g of compound of formula (IV), 29.41g of 1,3-cyclohexanedione, 27.83g of triethylamine, 3g of bis[(4-N,N-dimethylamino)phenyl]di-tert-butylphosphine palladium(II) dichloride, 250g of dioxane solvent were added to a high-pressure reactor. Replaced with nitrogen 3 times, then introduced CO at a pressure 1~4 MPa, heated and reacted at 85°C for about 3 hours. After confirming the completion of the reaction by HPLC monitoring, cooled to room temperature, released the pressure, opened the kettle, poured out the reaction solution, filtered, the filter cake was washed with 10 g of dioxane, the mother liquor was rotary evaporated to remove, added 80 g of ethanol to the spin-dried mother liquor, heated to 70 °C to dissolve, slowly cooled to crystallizate, filtered, and dried the solid at 70 °C to obtain (VIII) ester compounds, yield 71.63%.

**Method 2:** The operation is the same as Method 1 except for replacing the solvent with 300g of ethylene glycol diethyl ether

HPLC conditions: Same as in Example 5.

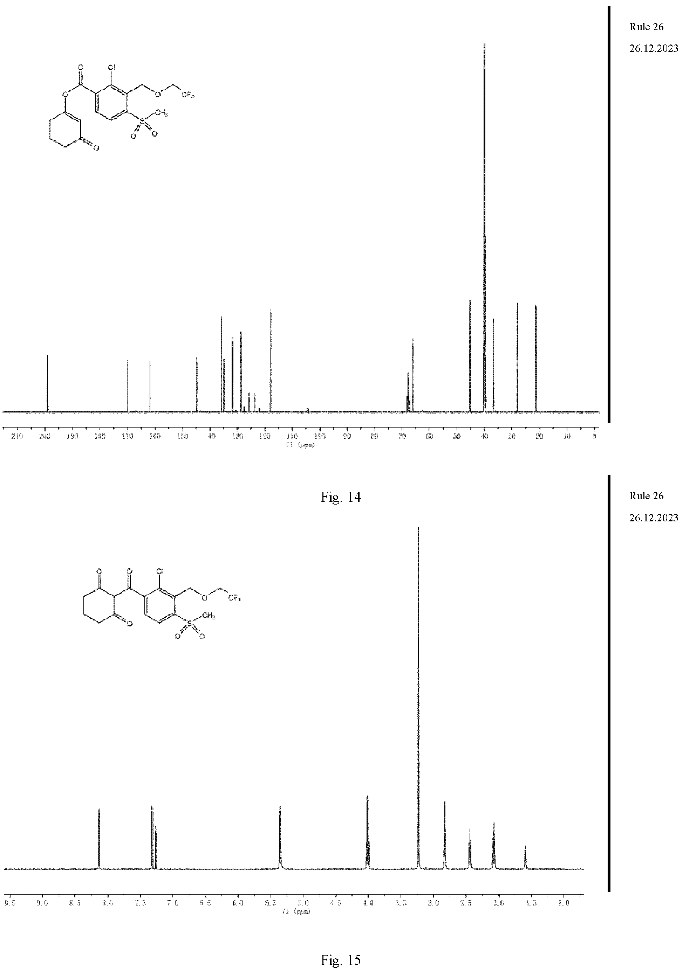

The ¹HNMR (DMSO) spectrum of the compound of formula (VIII) is shown in Figure 13, and the ¹³CNMR (DMSO) spectrum is shown in Figure 14.

### Example 10 Preparation of cyclosulfonone (II) (One-pot method)

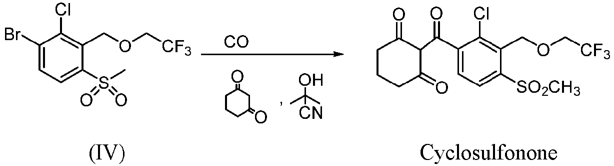

56.84g of the compound of formula (IV), 37.95g of triethylamine, 0.4g of PdCl₂ catalyst, 3.98g of 1,4-bis(diphenylphosphino)butane ligand, and 300g of 1,4-dioxane solvent were added to a high-pressure reactor. Evacuated and replaced with nitrogen three times, introduced CO at a pressure of about 1~4 MPa, and reacted at 80~85°C for about 8 hours, then cooled to 70°C, 2g of acetone cyanohydrin was added, and continue the reaction for 5 hours. After confirming the completion of the reaction by HPLC monitoring, cooled to room temperature, released the pressure, opened the reactor, filtered to remove triethylamine hydrobromide, rotary evaporated to remove the dioxane solvent, dissolved in methanol-water, adjusted the pH to 8~9 with sodium hydroxide solution, heated to 50°C with stirring, filtered out insoluble matter, adjusted the pH to 2~3 with acid, precipitated a white solid, the solution was cooled to room temperature and stirred in an ice bath for 0.5 hours, filtered, washed with water, filtered, and dried at 60°C to obtain cyclosulfonone as a yellow-white powder solid, yield 64.67%, melting point 120~122°C.

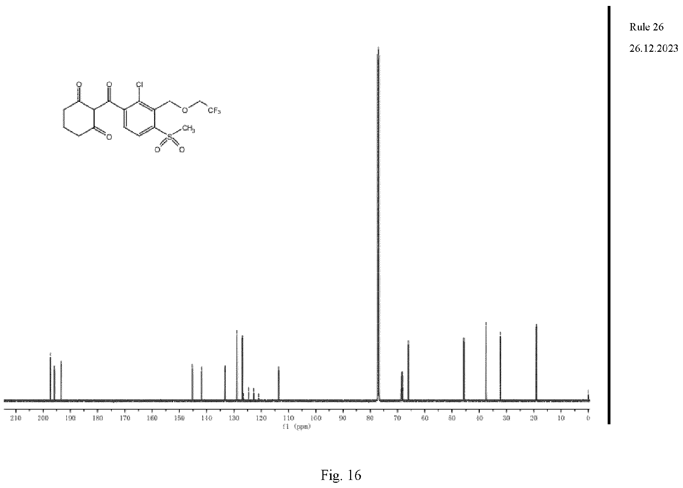

The ¹HNMR (DMSO) spectrum of the obtained cyclosulfonone is shown in Figure 15, and the ¹³CNMR (DMSO) spectrum is shown in Figure 16.

### Example 11

56.84g of the compound of formula (IV), 21.84g of 1,3-cyclohexanedione, 37.95g of triethylamine, 0.5g of PdCl₂ catalyst, 4g of 2-dicyclohexylphosphino-2',6'-dimethoxybiphenyl or 4,5-bis(diphenylphosphino)-9,9-dimethylxanthene ligand, and 290g of 1,4-dioxane solvent were added to a high-pressure reactor. Evacuated and replaced with nitrogen three times, introduced CO at a pressure of 1~4 MPa, and reacted at 85°C for about 10 hours. The yield was less than 10%, which was unsatisfactory.

## Claims

1. A method for preparing cyclosulfonone, which is **characterized by** comprising any one of the following steps:

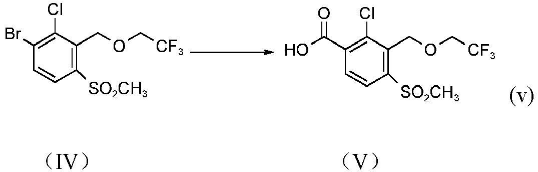

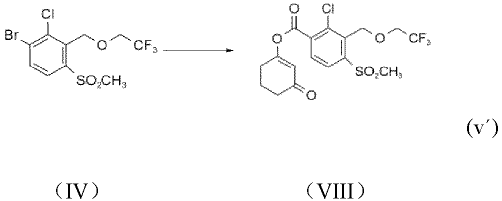

2. The method as claimed in claim 1, wherein steps (v) and (v') are respectively the following steps:

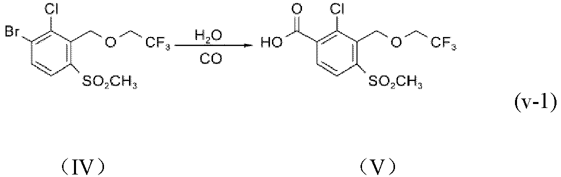

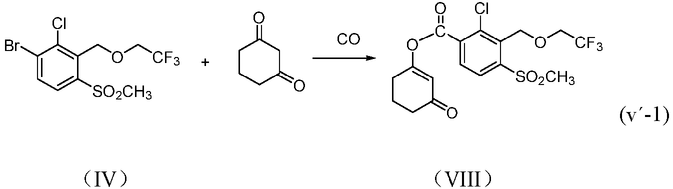

3. The method as claimed in claim 2, wherein the reaction of step (v-1) or (v'-1) is carried out in the presence of a noble metal catalyst and a phosphorus ligand or a salt thereof, the noble metal is selected from palladium, rhodium, ruthenium, copper, and cobalt, preferably a palladium catalyst, more preferably palladium chloride (PdCl₂), palladium carbonate (PdCO₃), palladium acetate[Pd(OAc)₂], palladium/carbon (Pd/C), palladium trifluoroacetate [Pd(OTFA)₂], or palladium bis(dibenzylideneacetone) [Pd(dba)₂];
the phosphorus ligand or a salt thereof is preferably 1,3-bis(diphenylphosphino)propane, 1,4-bis(diphenylphosphino)butane, or bis[(4-N,N-dimethylamino)phenyl]di-tert-butylphosphine.

4. The method as claimed in claim 3, wherein the reaction solvent is one or two or more selected from toluene, 1,4-dioxane, ethylene glycol dimethyl ether, ethylene glycol diethyl ether, dimethyl sulfoxide (DMSO), tetrahydrofuran (THF), and N,N-dimethylformamide (DMF).

5. The method as claimed in any one of claims 1 to 3, further comprising any one or more of the intermediate preparation steps (i) ~ (iv) as shown below:

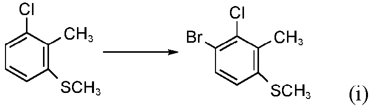

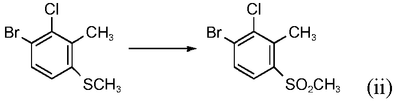

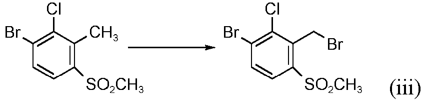

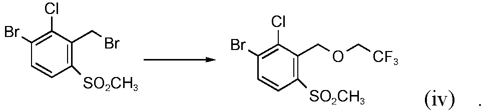

6. The method as claimed in claim 4, wherein the steps (i) to (iv) are respectively the following steps: 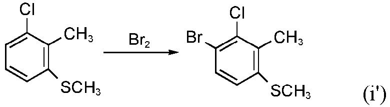 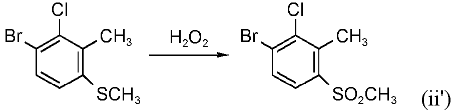 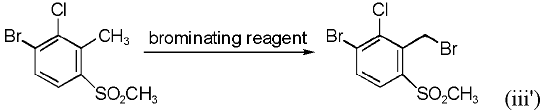 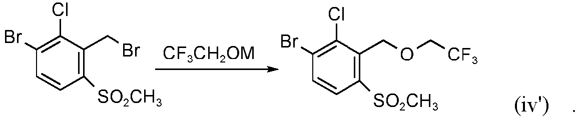
wherein in step (iii'), the brominating reagent is bromine, N-bromosuccinimide, or a combination of hydrogen bromide and hydrogen peroxide, preferably bromine;
wherein in step (iv'), CF₃CH₂OM is CF₃CH₂ONa or CF₃CH₂OK.

7. The method as claimed in any one of claims 1 to 6, comprising the following intermediate preparation steps:

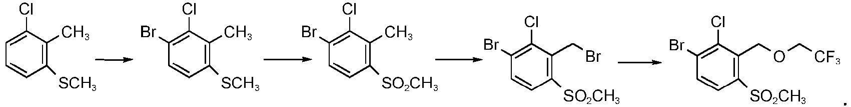

8. The method as claimed in claim 7, wherein the intermediate preparation steps are as follows: 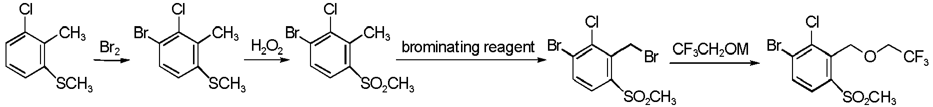 wherein in the formula, the brominating reagent is bromine, N-bromosuccinimide, or a combination of hydrogen bromide and hydrogen peroxide, preferably bromine; CF₃CH₂OM is CF₃CH₂ONa or CF₃CH₂OK.

9. The method as claimed in claim 7 or 8, further comprising the following steps: 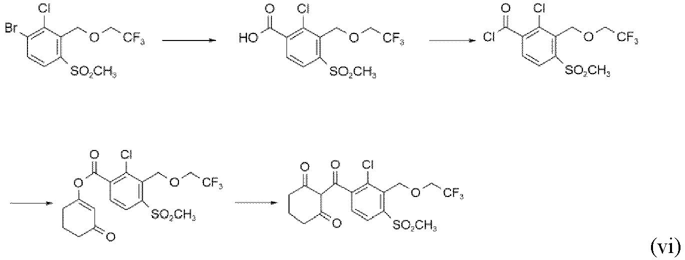 or 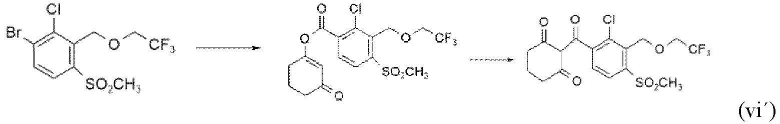 wherein step (vi') is carried out in a stepwise way with isolation of the intermediate ester compound or in a one-pot way without isolation of the intermediate ester compound.

10. The method as claimed in claim 9, wherein steps (vi) and (vi') are respectively the following steps: 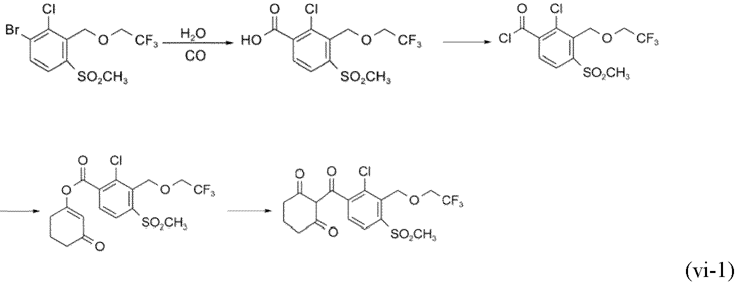 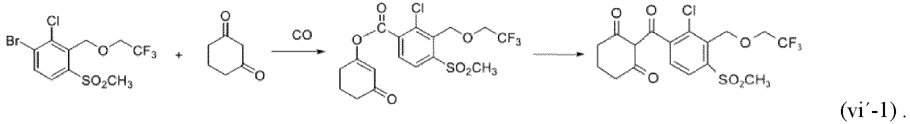 wherein step (vi'-1) is carried out in a stepwise way with isolation of the intermediate ester compound or in a one-pot way without isolation of the intermediate ester compound.

11. The compound shown in formula (A) as below: 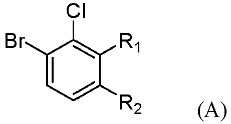 wherein R₁ and R₂ are any one of the following:
R₁ is CF₃-CH₂-O-CH₂-, and R₂ is CH₃-SO₂-;
R₁ is Br-CH₂-, and R₂ is CH₃-SO₂-;
R₁ is CH₃-, and R₂ is CH₃-SO₂-;
R₁ is CH₃-, and R₂ is CH₃-S-.

12. A method for preparing the compound shown in formula (A) as claimed in claim 11, comprising any one or two or more of the following steps (i) ~ (iv): 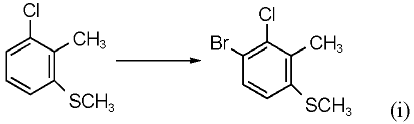 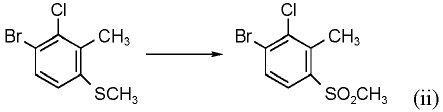 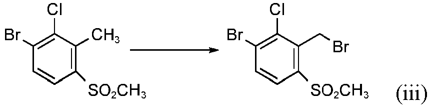 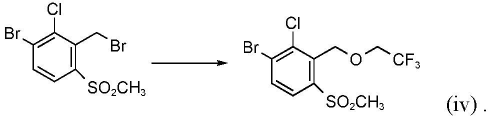 Preferably, steps (i) ~ (iv) are respectively the following steps: 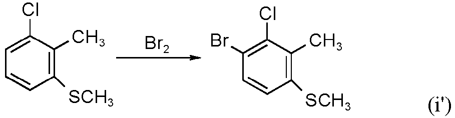 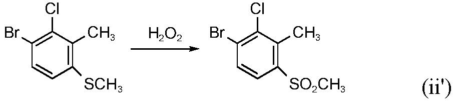 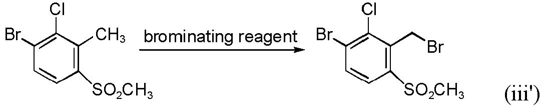 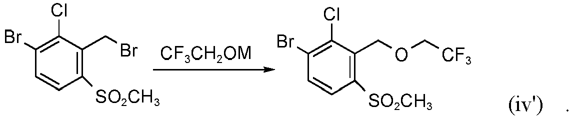
wherein in step (iii'), the brominating reagent is bromine, N-bromosuccinimide, or a combination of hydrogen bromide and hydrogen peroxide, preferably bromine;
wherein in step (iv'), CF₃CH₂OM is CF₃CH₂ONa or CF₃CH₂OK.

13. A method for preparing cyclosulfonone, which is **characterized by** using the compound according to claim 11 as an intermediate.

14. Use of the compound according to claim 11 as an intermediate for preparing cyclosulfanone.
